# EUROPEAN PATENT APPLICATION

(11) **EP 4 091 650 A1**
(43) Date of publication of application: **23.11.2022**
(21) Application number: 21175199.5
(22) Date of filing: 21.05.2021
(51) Int. Cl.: A61M 5/20, A61M 5/315

(54) **MONITORING UNIT FOR MONITORING INJECTION DEVICES**

(71) Applicant: Ypsomed AG, 3401 Burgdorf (CH)
(72) Inventor: Haberer, Christoph, 79346 Endingen (DE)
(74) Representative: Meier Obertüfer, Jürg

(57) **Abstract**

A monitoring unit (2) adapted to be attached to or provided as part of an injection device (1) for monitoring of a drug administration process executable by means of the injection device with a spring (3), in particular a helical spring, said spring comprising a first closed electrically conducting loop (13a) configured to move between a first start position and a first end position before, during and/or after the drug administration process; comprises:
i. a first inductive sensor;
ii. a second inductive sensor;
iii. a sensor drive unit for powering and/or controlling the inductive sensors, the sensor drive unit configured to induce a first circular electric current in the first closed electrically conducting loop;
iv. the first inductive sensor configured to provide a first response, in particular a first signal, to the first circular electric current which first response varies with a position of the first closed electrically conducting loop relative to the first inductive sensor;
v. the second inductive sensor configured to provide a second response, in particular a second signal, to the first circular electric current or to a second circular electric current induced in the first closed electrically conducting loop by the sensor drive unit, which second response varies with a position of the first closed electrically conducting loop relative to the second inductive sensor.

The monitoring unit is configured to determine and/or monitor a position and/or displacement of the first closed electrically conducting loop; wherein both the first and the second response are taken into account by the monitoring unit.

## Description

### SUMMARY OF THE INVENTION

The present invention relates to injection devices or medicament delivery devices for injecting, delivering, administering, infusing or dispensing flowable medical formulations, in particular comprising substances and/or liquids such as insulin or hormone preparations. It departs from a monitoring unit attachable to the injection device.

### TECHNICAL FIELD OF THE INVENTION

A variety of diseases exist that require regular treatment by subcutaneous administration of a medicament, and a number of drug delivery devices, in particular administration and/or application devices, have been developed to support a patient in accurately and controllably delivering a well-defined amount of drug in a self-administration process. Delivery devices include injection devices that are removed from the injection site after each medication event or drug delivery process, in particular drug administration and/or application process, as well as infusion devices with a cannula or needle that remains in the skin of the patient for a prolonged period of time. Disposable delivery devices are adapted to deliver, in particular administer or apply, a drug from a container such as a pre-filled syringe that is not intended to be replaced or refilled by the patient. Reusable, semi-reusable, or hybrid delivery devices have at least a container and possibly also a container holder that may be replaced by the patient, or a cartridge that may be refilled, while some components of the device may be reused with the replaced or refilled drug container. By way of example, diabetes may be treated by administration of insulin by the patients themselves with the help of multi-variable-dose insulin injection pens or infusion pumps.

In the present context, the terms "substance", "drug", "medicament" and "medication" are to be understood to include any flowable medical formulation suitable for controlled delivery, administration and/or application through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin- containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

Fixed dose disposable injection devices include single-dose injection devices such as auto injectors or patch injectors as well as multi-dose injection devices such as fixed dose injectors. Auto-injectors automatically deliver a fixed dose of liquid drug from a pre-filled syringe by means of a pre-tensioned injection spring biasing a piston rod and shifting a piston in a syringe barrel. Patch injectors or ready-to-use pre-filled wearable bolus injectors are patched or adhered to the skin of the patient in view of a single dose injection taking between thirty- seconds and several minutes. Fixed dose injectors have a single, non-variable dosage volume, or eventually provide a limited number of fixed, non-variable injection dosage volumes for the user to choose from.

Disposable delivery devices may be complemented by a monitoring unit adapted to be successively attached to the device housings of plural disposable delivery devices, wherein said monitoring unit may be part of a reusable electronic module or auxiliary device. The monitoring unit serves to monitor the drug delivery process and/or a drug delivery status, in order to proactively prevent or retroactively recognize false handling of the device and to keep track of the doses already applied. In addition to generating data related to an instantaneous status, condition, or use of the delivery device, information on the drug type, cartridge batch, and/or expiration date may be evaluated by the monitoring unit. To that end, the monitoring unit may comprise a delivery status sensing unit for tracking a progress of a medication event performed by means of the delivery device and/or for reading drug information that is stored on a machine-readable tag mounted to the device housing. The monitoring unit may further comprise a status indicator for signaling status and drug information to a user, and a wireless communication unit for communicating status and drug information to a nearby mobile device or medical gateway. All these units are supplied with electric power from an energy storing unit of the electronic module, wherein the electronic module generally excludes any kind of electrically powered mechanical actuator or motor load. An exemplary electronic module with a sensing unit capable of discerning various operational states of a disposable auto-injector is disclosed in PCT/CH2017/050004.

Alternatively, monitoring or control unit with the aforementioned sensor, indicator and communication functionalities may be an integral part of a reusable electronic delivery device and as such be integrated into a device housing of the delivery device comprising the reusable components. In this case, the electronic delivery device may be a reusable injection pen with a monitoring unit and a manually powered delivery drive requiring a user to manually provide the energy to move the piston or to charge a drive spring. The electronic delivery device may also be a reusable infusion pump with a monitoring unit and with a motor driving the piston automatically. All sensing, reading, evaluating, indicating, data processing, and communicating facilities of the monitoring unit are powered from an energy storing unit of the reusable delivery device.

The delivery status sensing unit generally detects a linear and/or rotational position and/or displacement of a movable component of the delivery device that occurs during a medication event. From the detected ongoing or completed displacement of the component a delivery status is inferred and communicated to a user. The definition of a delivery status in this context may be independent of certain aspects of component displacement, such as for instance a rotation direction of a screw-type plunger rod, or an absolute sensor signal amplitude. Accordingly, distinct types of delivery devices may well be characterized by the same formal delivery states, irrespective of the fact that certain inter-type variations in design geometry or in properties of the materials chosen may influence an unprocessed sensor signal.

The moveable component may be a helical spring which may be rigidly attached to and/or connected with a first component of the disposable delivery device near or at a first end of said helical spring, and to a second component of the disposable delivery device near or at a second end of said helical spring. The first and second component may be moveable relative to one another, in particular in a direction at least essentially parallel to a longitudinal axis of the helical spring, and cause the spring to be compressed or expanded when moving in such manner, wherein the first end of the spring will move relative to the second component and vice versa. The first and second component may be configured and/or required to move relative to one another during the delivery process, and may further both be configured and/or required to move relative to a third component of the disposable delivery device.

The delivery status sensing unit may be configured to detect a displacement and/or position of the first end and/or the second end of the helical spring relative to the first and/or second part of the disposable delivery device, in particular by means of a position sensor, in particular an inductive position sensor, as described in WO 2019/186412 A1, which is hereby included by reference in its entirety.

The position sensor may comprise an induction coil (also referred to as sensor induction coil), an inductance of which depends on and/or varies with a position of an inductive spring section relative to the induction coil due to a change in mutual inductance with position. The inductive spring section (referred to as conductive spring section in WO 2019/186412 A1) may involve at least two, preferably successive, winding turns of the helical spring that at least partially contact or overlap and thus allow for a circular electric current or an electric loop current to be induced and/or circulate in said inductive spring section. Such an inductive spring section may be formed anywhere along a helical spring (referred to as compressive spring or, shortly, spring in WO 2019/186412 A1), in particular at or near an end of the spring, where it may form or be formed by a base or foot of said helical spring, and wherein an electrical contact may be formed between at least a first turn of the winding and a second, adjacent winding turn at a point of overlap. Such an electrical contact may be established preferably by laser, spot, or resistive welding or soldering techniques applied to the first, second and possibly further turns of the winding at a point of contact or overlap.

The position sensor may thus be adapted to provide a continuous position sensor signal indicative of, in particular proportional to, an instantaneous or current position of the inductive spring section, in particular the first end and/or the second end of the helical spring relative to the second and/or first component of the disposable delivery device, respectively, and/or to the third component of the disposable delivery device, wherein the position sensor and/or the induction coil may, in particular, be provided at a specific position relative to the respective second, first and or third part of the disposable delivery device.

### SUMMARY OF THE INVENTION

One aspect that may need adequate attention to be paid to when determining a position of an inductive spring section as described above based on a changing and/or varying inductance as described above is that any change in an inductance provided by the helical spring, in particular an inductance of the inductive spring section, will also result in a change in inductance of the induction coil, which may otherwise be misinterpreted as a change in position of the inductive spring section relative to the induction coil.

While the inductance of the inductive spring section normally remains constant at least over very extended periods of time, i.e. over several months or even years, at least as long as a length and/or an expansion or compression state of the spring remains unchanged, it has been observed that such inductance may change dynamically if and/or while the spring is compressed or expanded, in particular if compression or expansion takes place rapidly, in particular on a time scale of a few to a few hundred milliseconds. This may, inter alia, be caused by the fact that two or more adjacent turns of the winding get temporarily in touch with one another, and/or one or more windings adjacent to the inductive spring section get temporarily in touch with inductive spring section, thus establishing an electric contact that will create a temporary, additional inductive spring section and/or change an inductance of the (existing) inductive spring section. In particular, a change an inductance of an existing inductive spring section will be caused by a temporary change of a number N of short circuited winding turns which establish the inductive spring section.

It is thus an object of the invention to allow for an accurate and reliable monitoring and/or determination of a position and/or a displacement of an inductive spring section as detailed above.

The above objective and other objectives are solved by a monitoring unit, system for administering a drug, and a method in accordance with the independent claims.

A monitoring unit, adapted to be attached to or provided as part of an injection device, in particular a disposable delivery device, for monitoring of a drug administration process executable by means of the injection device, may comprise:
a. a spring, in particular a helical spring, said spring comprising a first closed electrically conducting loop configured to move between a first start position and a first end position during and/or after the drug administration process; wherein
b. the monitoring unit may comprise:
   i. a first inductive sensor;
   ii. a second inductive sensor;
   iii. a sensor drive unit for powering and/or controlling the inductive sensors, the sensor drive unit configured to induce a first circular electric current in the first closed electrically conducting loop;
   iv. the first inductive sensor configured to provide a first response, in particular a first signal, to the first circular electric current which first response varies with a position of the first closed electrically conducting loop relative to the first inductive sensor, in particular a distance between the first closed electrically conducting loop and the first inductive sensor;
   v. the second inductive sensor configured to provide a second response, in particular a second signal, to the first circular electric current or to a second circular electric current induced in the first closed electrically conducting loop by the sensor drive unit, which second response varies with a position of the first closed electrically conducting loop relative to the second inductive sensor, in particular a distance between the first closed electrically conducting loop and the second inductive sensor; and
c. the monitoring unit may be configured to determine and/or monitor a position and/or displacement of the first closed electrically conducting loop; wherein both the first and the second response are taken into account by the monitoring unit.

The monitoring unit may be of the kind described above and/or in WO 2019/186412 A1.

The spring of the injection device may in particular be a helical spring, and may comprise an elastic wire or other electrically conducting material which may be wound in turns around an imaginary cylinder, cone etc. The spring may thus extend in a longitudinal direction, and have and/or define a longitudinal axis, which may at least essentially correspond to a longitudinal axis and/or axial direction of the imaginary cylinder, cone, etc., and may also at least essentially correspond to a second longitudinal axis of and/or a second longitudinal direction defined by the injection device.

The spring may be configured to at least temporarily bias and/or drive a first component of the injection device before, during or after the drug administration process, wherein the first component may move relative to a stationary component of the injection device, in particular in a direction at least essentially parallel to the longitudinal axis. The stationary component may in particular be provided at and/or form a proximal end of the injection device and may allow a user to hold and/or manipulate the injection device, in particular to place a distal end of the injection device onto a part of a body of the user or another subject. The stationary component may also allow for the monitoring unit or an electronic module containing the monitoring unit to be temporarily and/or reversibly attached to the injection device in and/or with a defined position and/or orientation relative to the stationary component as exemplary described in WO 2020/058875 A1, which is hereby included by reference in its entirety.

The first component may comprise an injection cannula or needle, which the spring, in particular a preloaded spring, may accelerate and/or drive to penetrate a skin of the user or another person. The spring may also drive a cover sleeve which may, in an original position or rest position, surround the needle, and in particular a tip thereof, to avoid injury to a user and/or other persons, on/or prevent contamination of the needle; wherein the spring may be loaded when the cover sleeve is moved from its original position or rest position to a retracted position to carry out an injection, and moved back under the influence of the spring after the drug administration process. By way of another example, the spring may drive a piston to displace a flowable medical formulation during the drug administration process.

At least one winding turn of the helical spring may be short circuited, in particular by an additional electric contact provided between a beginning and end of said turn, thus forming a first closed electrically conducting loop and/or constituting a first inductive spring section as described above. An additional electric contact of the kind mentioned may, in particular, result from a reduced local pitch of said at least one winding turn, in particular a reduced local pitch that equals or falls short of a diameter of the elastic wire or other electrically conducting material. Said short circuited winding turn constitutes a resonant electric circuit, which may in particular be represented by an RL-circuit comprising a resistance and an inductance connected in series in a closed loop or circuit. The short circuited winding turn may in particular be provided at and/or form an end or base of the spring, which in turn may be rigidly attached to and/or connected with the first component of the injection device.

The first electrically conducting loop may be configured to move between a first start position and a first end position before, during or after the drug administration process, in particular immediately before or immediately after the drug administration process. Monitoring a position and/or displacement of the first closed electrically conducting loop may thus allow to draw conclusions about the drug administration process, in particular about whether, and possibly when, the drug administration process has occurred, and also whether it was successful or unsuccessful, as exemplary described in WO 2019/186412 A1. The first start position may be different from the first end position, wherein a distance *d*_{SE,1} between first start position and first end position may be significantly smaller than a length *l* of the spring in longitudinal direction, in particular with *l* >> *d*_{SE,1}. Alternatively, the first start position may be identical with the first end position, with a different intermediate position, and with the first electrically conducting loop configured to move from the first start position to the first intermediate position, and from there back to the first end position equaling the first start position. In this case, a distance *d*_{SI,1} between first start position and first intermediate position may be significantly smaller than a length I of the spring in longitudinal direction, in particular with *l* >> *d*_{SI,1}. The length *l* of the spring may in particular be the length as measured in a non-tensioned, unbiased and/or relaxed state of the spring. Alternatively, it may also be the length of the spring as measured in an original state of the injection device prior to use, and/or with the first electrically conducting loop in the first start position, and preferably any further electrically conducting loops in their respective start position.

The monitoring unit may comprise first and second inductive sensors, which may be provided at a distance *d*₁₂ from one another. The monitoring unit may be temporarily and/or reversibly attached to the injection device, in particular to a stationary component of the injection device as described above. The distance *d*₁₂ may in particular extend at least essentially parallel to the longitudinal direction when the monitoring unit is attached to the injection device. The first and second inductive sensor may comprise a first and second induction coil, respectively, wherein each coil extends in a longitudinal sensor direction, and has and/or defines a longitudinal sensor axis, wherein each longitudinal sensor direction may extend in a direction at least essentially parallel to the longitudinal direction of the spring, and wherein the longitudinal sensor axis of each coil may at least essentially coincide with the longitudinal axis of the spring.

The first and second inductive sensor may, in particular with respect to the longitudinal direction of the spring, be located near the first start position and the first end position, respectively, or, where the first start position equals the first end position, near the first start position and the first intermediate position, respectively, wherein a position of the first and second inductive sensors may correspond to and/or be defined by a position of the first and second induction coil, respectively. In particular, the first inductive sensor may be located closer to the first start position than to the first end position or the first intermediate position, whereas the second inductive sensor may be located closer to the first end position or the first intermediate position than to the first start position. More specifically, the first inductive sensor may be provided at a location that at least essentially corresponds to the start position, and/or the second inductive sensor may be provided at a location that at least essentially corresponds to the first end position or the first intermediate position, all with respect to the longitudinal direction of the spring. The distance *d*₁₂ may be significantly smaller than the length *l* of the spring, i.e. *I > d*₁₂*,* in particular *l* >> *d*₁₂.

The sensor drive unit may be provided to power and/or control the inductive sensors, and may in particular be configured to selectively apply a time-dependent voltage and/or current to the first and/or second inductive sensor, in particular to the first and second induction coils, respectively, and/or to measure time-dependent voltages and/or currents at or in the first and/or second inductive sensor.

The sensor drive unit may thus provide for a first and/or second current to flow in the first and/or second induction coil, respectively, in particular for time-dependent first and second currents to alternatingly flow in the first and second induction coil. The first and/or second induction coil will thus act as first and/or second inductors, respectively, and the first and/or second currents will produce first and/or second magnetic fields, in particular magnetic flux densities ***B*₁** and ***B*₂**, within and in an environment of the respective coil, each which will also at least partially penetrate the other induction coil. The first and/or second currents may be time-dependent and may in particular be representable by a pulse or step function, or by an at least approximately sinusoidal function with fixed or varying frequency.

Alternatively, the first inductive sensor may further comprise a first generation coil, and the second inductive sensor may optionally comprise a second generation coil; and the sensor drive unit may be configured to apply a time-dependent voltage and/or current to the first generation coil and, if a second generation coil is present, to said second generation coil, preferably in an alternating manner. The first and/or second generation coils will thus act as first and/or second inductors, respectively, and the first and optional second currents will produce first and optional second magnetic fields, in particular magnetic flux densities ***B*₁** and ***B*₂**, within and in an environment of the respective generation coil, each which will also at least partially penetrate into each of the induction coils.

The magnetic flux densities ***B*₁** and ***B*₂** will also give rise to a flow of a circular electric current within the first closed electrically conducting loop, which generates a secondary magnetic field, in particular a secondary magnetic flux density ***B***_{sec}, which will also at least partially penetrate the first and/or second induction coil.

Under the influence of the circular electric current within the first closed electrically conducting loop, in particular the secondary magnetic flux density ***B***_{sec}, the first inductive sensor will provide a first response, in particular a first signal, to the first circular electric current, which first response will vary with a position of the first closed electrically conducting loop relative to the first inductive sensor.

The first response may be quantitatively described and/or characterized by a quantity *R*₁, which may in particular be a function of time. More specifically, the first response may, in particular, be a voltage across the first induction coil, or across a resistor connected in series with the first induction coil to form a closed electric circuit further containing a series-connected current source controlled or provided by the sensor drive unit.

Under the influence of the circular electric current within the first closed electrically conducting loop, in particular the secondary magnetic flux density ***B***_{sec}, the second inductive sensor will provide a second response, in particular a second signal, to the second circular electric current, which second response will vary with a position of the second closed electrically conducting loop relative to the second inductive sensor.

The second response may be quantitatively described and/or characterized by a quantity *R*₂, which may in particular be a function of time. More specifically, the second response may, in particular, be a voltage across the second induction coil, or across a resistor connected in series with the second induction coil to form a second closed electric circuit further containing the or a second series-connected current source controlled or provided by the sensor drive unit.

With any other factors which may influence the response unchanged and/or kept constant, *R*₁ will in general depend on a distance *d*₁ between the first induction coil, in particular an end of the first inducting coil facing the first closed electrically conducting loop and the first closed electrically conducting loop, wherein the response will decrease rapidly with distance. As a rule of thumb, the response will become too weak to be detected with reasonable effort and/or accuracy when the distance *d*₁ exceeds a diameter *D*_{ind} of the induction coil.

Likewise, with any other factors which may influence the response unchanged and/or kept constant, *R*₂ will in general depend on the distance *d*₂ between the second induction coil, in particular an end of the second inducting coil facing the first closed electrically conducting loop and the first closed electrically conducting loop, wherein the response will decrease rapidly with distance. As a rule of thumb, the response will become too weak to be detected with reasonable effort when the distance *d*₂ exceeds a diameter *D*_{ind} of the induction coil.

With any other factors which may influence the response unchanged and/or kept constant, the first and second responses *R*₁ and *R*₂ will in general also depend on the number *N* of short circuited winding turns which establish the first closed electrically conducting loop. In particular, for exemplary embodiments to be described further down, first and second responses *R*₁ and *R*₂ may at least approximately be proportional to said number N of winding turns.

First and second responses *R*₁ and *R*₂ may thus be described as functions of *d*₁ and *d*₂, respectively, as well as of N, i.e. *R*₁ = *R*₁ (*d*₁, *N*) and *R*₂ = *R*₂ (*d*₂*, N*), wherein a potential dependence on time is not explicitly been indicated for reasons of readability. First and second responses *R*₁ and *R*₂ may be regarded as raw responses, which may in particular be obtained by direct measurements of electric quantities like voltage or current. Said raw responses may be processed by the sensor drive unit or another unit comprised by the monitoring unit to obtain processed responses *R*'₁ and *R*'₂, which in turn may be described as functions of *d*₁ and *d*₂, respectively, as well as of *N*, i.e. *R'*₁ *= R*'₁ (*d*₁, *N*) and *R'*₂ *= R'*₂ (*d*₂, *N*). The processed responses may be provided in digital form. By way of example, they may represent an inductance of the first or second induction coil, respectively, which may respectively depend on a distance *d*₁ between the first induction coil, in particular an end of the first inducting coil facing the first closed electrically conducting loop and the first closed electrically conducting loop or on a distance *d*₂ between the second induction coil, in particular an end of the second inducting coil facing the first closed electrically conducting loop and the first closed electrically conducting loop, respectively.

For a given type of injection device, monitoring unit, first and second induced circular electric current, and/or other aspects, *R*₁ *= R*₁ (*d*₁, *N*), *R*₂ *= R*₂ (*d*₂, *N*), *R*'₁ (*d*₁*, N*) and/or *R'*₂ = *R'*₂ (*d*₂, *N*) may be determined in an analytic manner, and/or by sampling responses over a number of exemplary values for *d*₁, *d*₂, and *N*, in particular for prototype devices and/or monitoring units, and by subsequently fitting a function and/or populating a look-up table.

If and as long as ***N*** is known and may be considered constant, i.e. *N* = *N*₀, the position of the first closed electrically conducting loop along the longitudinal axis of the spring between the first and second induction coil may either be determined or at least estimated exclusively based on *R*₁ (*d*₁, *N*₀) and/or *R*'₁ (*d*₁, *N*₀), or exclusively based on *R*₂ (*d*₂, *N*₀) and/or *R'*₂ (*d*₂, *N*₀), wherein it may further be taken into account that *d*₂ = *d*₁₂ - *d*₁. Such determination or estimation of the position using response from one inductive sensor only may be carried out according to the principles laid down in WO 2019/186412 A1 with respect to each of the first and second cover sleeve spring bases mentioned therein. However, if ***N*** varies or changes unnoticedly so that ***N ≠** N*₀, a wrong position of the first closed electrically conducting loop may be obtained under this approach.

Such an erroneous determination of the position of the first closed electrically conducting loop may be detected if the monitoring unit is configured to take both the first and the second response into account when determining and/or monitoring the position, e.g. by carrying out plausibility checks to verify that the position has been correctly determined. Such a plausibility check may, by way of example, be based on the fact that when the first electrically conducting loop moves away from one (e.g. the first) inductive sensor towards the other (e.g. the second) inductive sensor, the first response may be expected to weaken and/or decrease, whereas the second response may be expected to get stronger and/or increase (and vice versa); whereas when the number the number *N* of short circuited winding turns which establish the inductive spring section changes while the position remains essentially constant, both the first and second responses may be expected to change in an analogous manner, i.e. they may be expected to either both weaken and/or decrease, or both get stronger and/or increase. Thus, a correlation or anti-correlation - or a lack thereof - between the first and second response may exemplarily be employed in order to take both responses into account in determining and/or monitoring the position.

Alternatively or in addition, a ratio and/or a difference between the first and the second response may be taken into account in monitoring the position and/or displacement of the first closed electrically conducting loop.

By way of example, based on the consideration as laid out above and regarding the different expected relationships between first and second response under displacement of the first electrically conducting loop moves and changes of ***N,*** respectively, it may be assumed that the first closed electrically conducting loop remains at rest as long as a modulus of a difference between first and second response remains below a predetermined first threshold, and/or a ratio between first and second response or second and first response remains below a predetermined second threshold.

In embodiments, by taking into account both one of *R*₁ and *R*'₁ and one of *R*₂ and *R*'₂, the position of the first closed electrically conducting loop along the longitudinal axis of the spring between the first and second induction coil may be determined or at least estimated along with the number N of short circuited winding turns, wherein it may further be taken into account that *d*₂ = *d*₁₂ - *d*₁, and/or that N may only assume integer values. This may be achieved in a manner analogous to solving multiple equations with multiple unknowns: In a first step, an instantaneous and/or measured first response *R̂*₁ and/or *R̂*₁' may be compared with previously determined functions *R*₁ (*d*₁, *N*) and/or *R*'₁ (*d*₁, *N*) to obtain plural pairs of values *d̂*₁ and *N̂* for which *R̂*₁ *= R*₁ (*d*₁*, N̂*) and/or *R̂*ₜ' = *R*'₁ (*d*₁*, N̂*) holds at least approximately. By subsequently examining, with *d̂*₂ = *d*₁₂ - *d̂*₁ for each pair of values, for which of said pairs of values *R̂*₂ = *R*₂ (*d*₂, *N̂*) and/or *R̂*₂' = *R'*₂ (*d̂*₂, *N̂*) is fulfilled or at least approximated with the smallest deviation, correct values for *d*₁, *d*₂, and *N* may be obtained.

The first inductive sensor and the second inductive sensor may, together with the sensor drive unit and optional evaluation unit, configured to determine the position of the first closed electrically conducting loop following the principles laid down above, constitute a position sensor, in particular of the kind described in WO 2019/186412 A1, configured to provide a continuous position sensor signal indicative of an instantaneous or current position of a first component of the injection device, wherein an error due to variations and/or fluctuations in the number *N* of short circuited winding turns has been eliminated. The monitoring unit may further comprise a position discriminator and a status evaluator or state estimator, each of the kind described in WO 2019/186412 A1.

A system for administering a drug may comprise:
a. an injection device, said injection device comprising:
   i. a spring, in particular a helical spring, said spring comprising a first closed electrically conducting loop configured to move between a first start position and a first end position during or after the injection process;
b. a monitoring unit as described above, said monitoring unit attached to or provided as part of the injection device.

The injection device may in particular be of the kind described in detail above. The monitoring unit may be temporarily and/or reversibly attached to the injection device, in particular if the injection device is a disposable or single use injection device. Alternatively, the monitoring unit may be permanently connected with the injection device, in particular provided as part of the injection device and/or formed integrally with the injection device, in particular if the injection device is configured to be used multiple times, wherein a flowable medical formulation may be replenished after each use or after several drug delivery processes.

A method of monitoring a drug administration process executed by means of the injection device, with said injection device comprising:
i. a spring, in particular a helical spring, said spring comprising a first closed electrically conducting loop configured to move between a first start position and a first end position during or after the injection process;
may comprise the steps of:
i. providing a first inductive sensor in proximity to the first start position;
ii. providing a second inductive sensor in proximity to the first end position or the intermediate position;
iii. inducing a first circular electric current in the first closed electrically conducting loop to generate a first response, in particular a first signal, that varies with a position of the first closed electrically conducting loop relative to the first inductive sensor;
iv. generating a second response, in particular a second signal, that varies with a position of the first closed electrically conducting loop relative to the second inductive sensor;
v. receiving the first and second response;
vi. monitoring a position and/or displacement of the first closed electrically conducting loop, wherein both the first and the second response are taken into account.

The method may, in particular, be carried out by a monitoring unit as described above attached to the injection device.

Preferred embodiments and/or variations of the invention are presented in dependent claims.

### BRIEF DESCRIPTION OF THE DRAWINGS

Below, the subject matter of the invention will be explained with respect exemplary embodiments which are illustrated in the attached drawings, of which
Fig 1 shows an exemplary an exemplary electronic module comprising a monitoring unit in accordance with the invention;
Fig 2 shows a flow chart of the pertinent blocks of the monitoring unit;
Fig. 3 illustrates an exemplary physical detection principle that may be employed in obtaining a continuous position sensor signal;
Fig. 4 shows selected features of an exemplary monitoring unit in accordance with the invention;
Fig. 5 shows selected features of another exemplary monitoring unit in accordance with the invention.

### DETAILED DESCRIPTION OF EXEMPLARY EMBODIMENTS

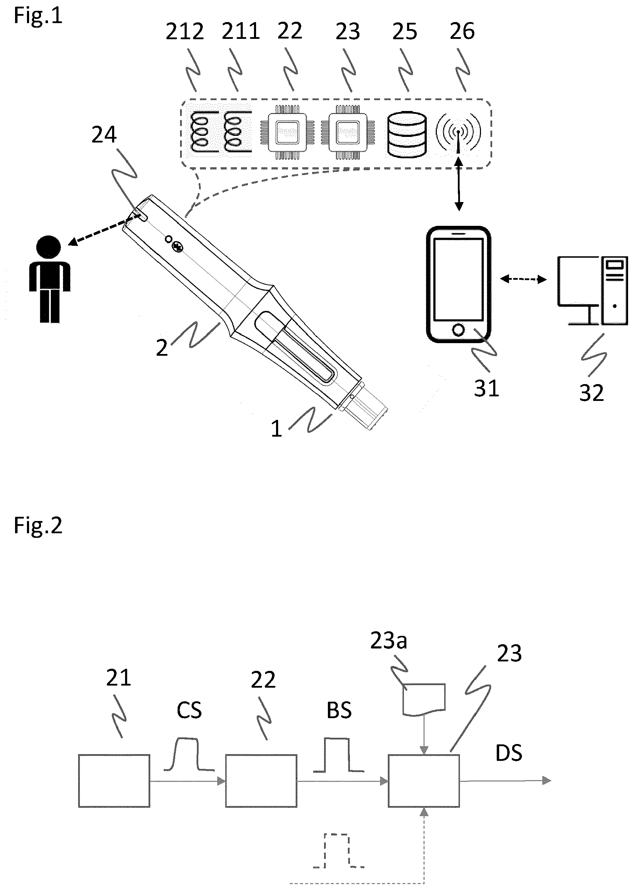

Fig.1 depicts an exemplary electronic module 2 which is releasably attached to an injection device 1 comprising a spring comprising a first closed electrically conducting loop, in particular a helical spring, as described above. The electronic module 2 includes a monitoring unit in accordance with the invention as hereinafter claimed, comprising, inter alia, a first inductive sensor 211 and a second inductive sensor 212. The electronic module further comprises a position discriminator 22, a status evaluator 23, and a status indicator 24 such as a LED, buzzer, vibration alarm, or any other type of HMI element for providing visual, acoustic, or tactile feedback about a derived injection status. A memory or data storage unit 25 is adapted to store status or delivery information.

The electronic module may comprise or represent a logic circuit. The electronic module may comprise an integrated circuit, in particular a general purpose central processing unit (CPU), a graphics processing unit (GPU), a microcontroller, a reduced instruction set computer (RISC) processor, an application specific integrated circuit (ASIC), a programmable logic circuit (PLC), a field programmable gate array (FPGA), a digital signal processing (DSP) device, and/or any other circuit or processing device capable of providing and/or executing functionalities described above and/or below.

The electronic module also comprises a communication unit 26 for wireless transmission of an injection status or drug status to the mobile device via Bluetooth Low Energy (BTLE) or equivalent short or near range wireless communication technology, which may be used for communication, in particular in particular data exchange, with a mobile device 31 such as a smartphone or tablet device running a dedicated application program; or a laptop computer configured accordingly. The mobile device may in turn be communicatively connected via a data communication network, e.g. the Internet, to a remote sewer, cloud based computing facility, or expert system 32. The electronic module 2 has a rear, or proximal, part where some or all electronic components as described are located.

Fig. 2 depicts a flow chart of the pertinent blocks of the monitoring unit, of which the position sensor 21 provides a continuous position sensor signal CS to the position discriminator 22 that in turn generates a binary position signal BS based on the continuous position signal. The binary position signal is evaluated, together with optional further binary position signals (dotted lines), by the status evaluator 23, based on an evaluation table 23a coding the delivery states as a combination of binary position signal values, to identify a delivery state DS.

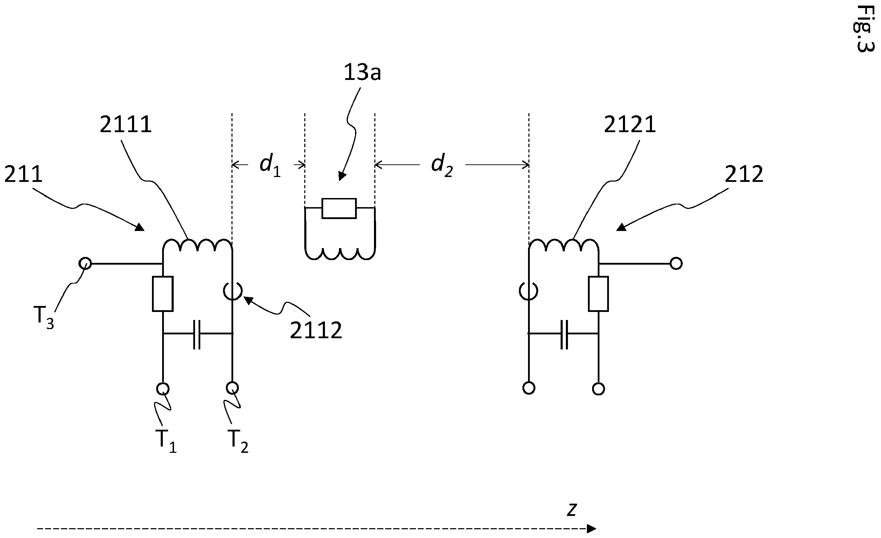

Fig. 3 illustrates an exemplary physical detection principle that may be employed in obtaining the continuous position sensor signal. The first inductive sensor 211 may thus comprise a first induction coil 2111 connected in series with a resistor and in parallel with a capacitance, wherein resistor and/or capacitance may be parasitic. A resulting first RL or RLC resonant circuit may be excited by a voltage source or current source connected between terminals T₁ and T₂. Oscillation of the resonant circuit may be monitored by measuring a voltage at the first induction coil 2111 or the resistor using terminals T₂ / T₃ or T₁ / T₃, respectively, or by measuring a current in the resonant circuit using an optional current sensor 2112.

Due to inductive coupling, a first closed electric loop 13a formed at a first end of the spring and provided at a distance *d*₁ from the first induction coil 2111 along a longitudinal direction *z* will influence a first resonant frequency *f*₁ of the first resonant circuit, wherein *f*₁ will depend on the distance *d*₁, i.e. *f*₁ = *f*₁ (di), at least as long as *d*₁ does not exceed a diameter *D*_{ind} of the first induction coil 2111. If the first closed electric loop 13a is constituted by short circuited winding turns of a helical spring as described further above, the first resonant frequency *f*₁ will also change with a number *N* of such short circuited winding turns, so that *f*₁ *= f*₁ (*d*₁, *N*). The first resonant frequency *f*₁ (*d*₁, *N*), which may be determined in various manners known to a person skilled in the art, may thus serve as a first response *R*₁, as described further above, in particular as processed first response *R*'₁.

Analogous considerations apply to the second inductive sensor 212 and a second induction coil 2121 comprised by said second inductive sensor 212, wherein a second resonant frequency *f*₂ (*d*₂, *N*) may serve as a first response *R*₂, as described further above, in particular as processed first response *R*'₂.

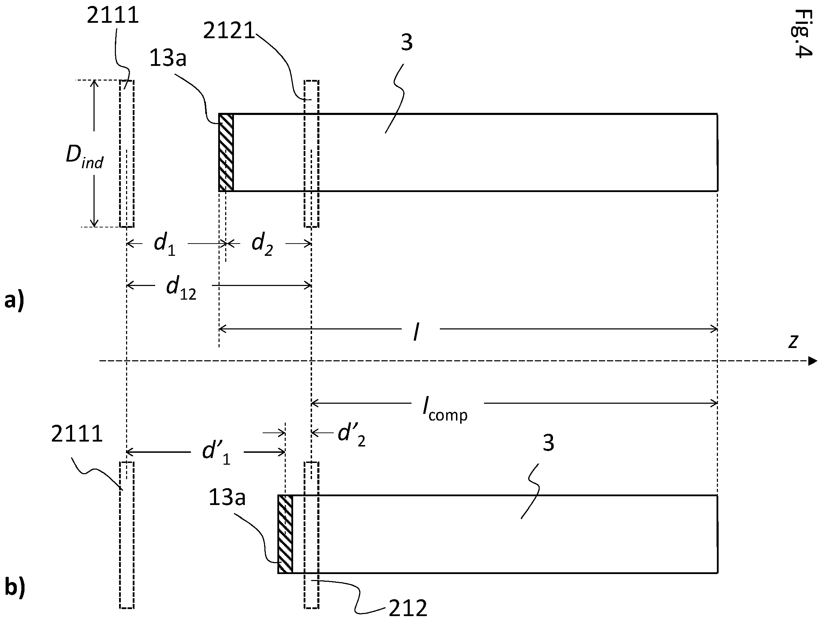

Fig. 4a shows selected features of an exemplary monitoring unit in accordance with the invention attached to or provided as part of an injection device of the type described above to illustrate an exemplary arrangement of the spring 3 of the injection device having a length *l*, with first closed electric loop 13a formed at a first end of the spring 3 in a first start position and of first and second induction coils 2111 and 2121, wherein *d*₂ ≈ *d*₁ *≈ d*₁₂*.*

Fig. 4b shows the arrangement of Fig. 4a, albeit with first closed electric loop 13a in a first end position, with *d*'₂ < *d*'₁, and with the spring compressed to a length *l*_{comp}.

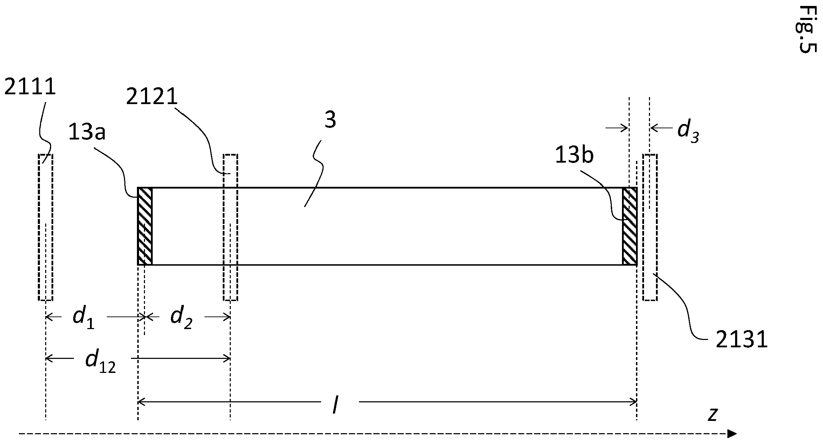

Fig. 5 shows selected features of another exemplary monitoring unit in accordance with the invention attached to an injection device of the type described above. Said monitoring unit comprises a third inductive sensor with a third induction coil 2131. A second closed electric loop 13b is formed at a second end of the spring 3, and located in proximity of third induction coil 2131 when in a second start position, wherein for a distance *d*₃ between the second closed electric loop 13b in a start position and third induction coil 2131, *d*₃ « *l*. The third inductive sensor may be employed to monitor a position and/or displacement of the second closed electric loop 13b, and thus of a second component of the injection device rigidly connected to second end of the spring 3, in a manner analogous to the one outlined further above for the first closed electric loop 13a using only the first inductive sensor.

This description and any accompanying drawings that illustrate aspects and embodiments of the present invention should not be taken as limiting the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the spirit and scope of this description and the claims. In some instances, well-known circuits, structures and techniques have not been shown in detail in order not to obscure the invention. Thus, it will be understood that changes and modifications may be made by those of ordinary skill within the scope and spirit of the following claims. In particular, the present invention covers further embodiments with any combination of features from different and/or individual embodiments as described above and below. Embodiments in accordance with the invention may, in particular, include further and/or additional features, elements, aspects, etc. not shown in the drawings or described above.

The disclosure also covers all further features shown in any Figure, individually, although they may not have been described in the afore or following description. Also, individual alternatives of the embodiments described in any Figure and the description and individual alternatives of features thereof can be disclaimed from the subject matter of the invention or from disclosed subject matter. The disclosure comprises subject matter consisting of the features defined in the claims or the exemplary embodiments as well as subject matter comprising said features.

The present disclosure also includes embodiments with any combination of features which are mentioned or shown above and/or below, in various embodiments or variants. It also includes individual features as shown in the Figures, even if they are shown there in connection with other features and/or are not mentioned above or below. The disclosure comprises embodiments which exclusively comprise the features described in the claims or the exemplary embodiments, as well as those which comprise additional other features. The steps of any method disclosed above or claimed below may preferably be carried out according the order in which they are presented, but may also be carried out in a different order.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "substantially", "about", "approximately" and the like in connection with an attribute or a value particularly also define exactly the attribute or exactly the value, respectively. The term "about" in the context of a given numerate value or range may refer to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

Unless stated otherwise, it shall be assumed throughout this entire document that a statement *a* ≈ *b* may imply that |*a*-*b*|/(|*a*|+|*b*|) < 0.2, preferably |*a*-*b*|/(|*a*|+|*b*|) < 0.05, wherein a and *b* may represent arbitrary quantities, parameters and/or variables as described and/or defined anywhere in this document, or as otherwise known to a person skilled in the art. Further, a statement that *a* is at least approximately equal or at least approximately identical to *b* may imply that *a* ≈ *b,* and not exclude that *a* = *b.* Further, unless stated otherwise, throughout this entire document, a statement *a* >> *b* may imply that *a > 5b,* preferably *a > 10b;* and statement *a* << *b* may imply that *5a* < *b,* preferably 10*a* < *b.*

Embodiments of the invention may involve one or more electronic or computing devices, and/or involve the use of such devices. Said devices typically include a processor, processing device, or controller, such as a general purpose central processing unit (CPU), a graphics processing unit (GPU), a microcontroller, a reduced instruction set computer (RISC) processor, an application specific integrated circuit (ASIC), a programmable logic circuit (PLC), a field programmable gate array (FPGA), a digital signal processing (DSP) device, and/or any other circuit or processing device capable of executing the functions described herein. In particular, the sensor drive unit, the optional evaluation unit, the position discriminator, and/or the status evaluator may be partially or fully implemented by or on such electronic or computing devices, individually or jointly.

The methods described herein may be partially or fully implemented as or in the form of software, which may in turn be encoded as executable instructions embodied in a non-transitory computer readable medium, including, without limitation, a storage device and/or a memory device. Such instructions, when executed by a processing device, cause the processing device to perform at least a portion of the methods described herein, preferably in real-time. The above examples are exemplary only, and thus are not intended to limit in any way the definition and/or meaning of the term processor and processing device.

As used herein, i.e. anywhere in this document, the terms "computer", "computing device", "smartphone", and related terms, e.g., "processor", "processing device," central processing unit (CPU)", "controller" and/or "control unit" may not be limited to just those integrated circuits referred to in the art as a computer, but broadly refer to a microcontroller, a microcomputer, a programmable logic controller (PLC), and application specific integrated circuit, and other programmable circuits, and these terms are used interchangeably herein. In the embodiments described herein, memory may include, but is not limited to, a computer-readable medium, such as a random access memory (RAM), a computer-readable non-volatile medium, such as a flash memory. Alternatively, a floppy disk, a compact disc - read only memory (CD-ROM), a magneto-optical disk (MOD), a digital versatile disc (DVD), a USB stick and/or a flash memory card (e.g. CF, SD, miniSD, microSD) may also be used.

## Claims

1. A monitoring unit (2) adapted to be attached to or provided as part of an injection device (1) for monitoring of a drug administration process executable by means of the injection device, said injection device comprising:
a. a spring (3), in particular a helical spring, said spring comprising a first closed electrically conducting loop (13a) configured to move between a first start position and a first end position before, during and/or after the drug administration process; wherein
b. the monitoring unit (2) comprises:
i. a first inductive sensor;
ii. a second inductive sensor;
iii. a sensor drive unit for powering and/or controlling the inductive sensors, the sensor drive unit configured to induce a first circular electric current in the first closed electrically conducting loop;
iv. the first inductive sensor configured to provide a first response, in particular a first signal, to the first circular electric current which first response varies with a position of the first closed electrically conducting loop relative to the first inductive sensor;
v. the second inductive sensor configured to provide a second response, in particular a second signal, to the first circular electric current or to a second circular electric current induced in the first closed electrically conducting loop by the sensor drive unit, which second response varies with a position of the first closed electrically conducting loop relative to the second inductive sensor; and
c. the monitoring unit is configured to determine and/or monitor a position and/or displacement of the first closed electrically conducting loop; wherein both the first and the second response are taken into account by the monitoring unit.

2. The monitoring unit (2) of claim 1, wherein the sensor drive unit is further configured to take a correlation between the first and the second response into account in monitoring the position and/or displacement of the first closed electrically conducting loop.

3. The monitoring unit (2) of claim 1, wherein the sensor drive unit is further configured to take a ratio of and/or a difference between the first and the second response into account in monitoring the position and/or displacement of the first closed electrically conducting loop.

4. The monitoring unit (2) of any preceding claim, further comprising
a. a third inductive sensor, wherein
b. the sensor drive unit or a second sensor drive unit is configured to
i. induce a third circular electric current in a second closed electrically conducting loop (13b) of the spring at a distance from the first electrically conducting loop (13a), and
c. the third inductive sensor is configured to provide a third response, in particular a third signal, to the third circular electric current that varies with a position of the second closed electrically conducting loop relative to the third inductive sensor, wherein
i. the monitoring unit is further configured to monitor a position and/or displacement of the second closed electrically conducting loop, wherein
ii. the third response is taken into account by the monitoring unit, and wherein
iii. the first and second responses are preferably not taken into account by the monitoring unit; and wherein
iv. the third response is preferably not taken into account in monitoring the position and/or displacement of the first closed electrically conducting loop (13a).

5. The monitoring unit (2) of any preceding claim, wherein
a. the first inductive sensor comprises a first inductor for inducing the first circular electric current in the first closed electrically conducting loop and/or the second circular electric current in the second closed electrically conducting loop,
b. the sensor drive unit is configured to energize the first inductor to induce the circular electric current or currents.

6. The monitoring unit of any preceding claim, wherein
a. the second inductive sensor comprises a second inductor for inducing the second circular electric current in the first closed electrically conducting loop,
b. the sensor drive unit is configured to energize the second inductor to induce the second circular electric current.

7. A system for administering a drug, said system comprising
a. an injection device (1), said injection device comprising:
i. a spring (3), in particular a helical spring, said spring comprising a first closed electrically conducting loop configured to move between a first start position and a first end position during or after the injection process;
b. a monitoring unit (2) according to one of claims 1 to 6 attached to or provided as part of an injection device (1).

8. The system according to claim 7, wherein a distance *d*₁₂ between the first and the second inductive sensor is smaller than a length *l* of the spring in a non-tensioned, unbiased and/or relaxed state, preferably with *d*₁₂ < 0.5 *l*, most preferably *d*₁₂ < 0.2 *l*.

9. The system according to claim 7 or 8, wherein a distance *d*₁₂ between the first and the second inductive sensor is smaller than a distance, in particular a minimum distance *l*₁₂ between the first closed electrically conducting loop and the second closed electrically conducting loop, preferably with *d* < 0.5 *l*₁₂, most preferably *d* < 0.2 *l*₁₂.

10. A method of monitoring a drug administration process executed by means of an injection device (1),
a. said injection device comprising:
i. a spring (3), in particular a helical spring, said spring comprising a first closed electrically conducting loop (13a) configured to move between a first start position and a first end position before, during or after the injection process;
b. the method comprising the steps of
i. providing a first inductive sensor in proximity to the first start position;
ii. providing a second inductive sensor in proximity to the first end position and/or the first intermediate position;
iii. inducing a first circular electric current in the first closed electrically conducting loop to generate a first response, in particular a first signal, that varies with a position of the first closed electrically conducting loop relative to the first inductive sensor;
iv. generating a second response, in particular a second signal, that varies with a position of the first closed electrically conducting loop relative to the second inductive sensor;
v. receiving the first and second response;
vi. monitoring a position and/or displacement of the first closed electrically conducting loop, wherein both the first and the second responses are taken into account.

11. The method of claim 10, further comprising inducing a second circular electric current in the first closed electrically conducting loop to generate a second response.

12. The method of claim 11, wherein
a. the first circular electric current in the first closed electrically conducting loop is induced using the first inductive sensor;
b. the second circular electric current in the first closed electrically conducting loop using the second inductive sensor.

13. The method of one of claims 10 to 12, wherein a correlation between the first and the second response is taken into account in monitoring the position and/or displacement of the first closed electrically conducting loop.

14. The method of one of claims 10 to 12, wherein a ratio of and/or a difference between the first and the second response is taken into account in monitoring the position and/or displacement of the first closed electrically conducting loop.

15. The method of one of claims 10 to 14, wherein
a. the spring (3) comprises a second closed electrically conducting loop (13b) configured to move between a second start position and a second end position before, during or after the injection process;
b. the method further comprising the steps of:
i. providing a third inductive sensor in proximity to the second start position;
ii. inducing a third circular electric current in the second closed electrically conducting loop to generate a third response, in particular a third signal, that varies with a position of the second closed electrically conducting loop relative to the third inductive sensor;
iii. receiving the third response;
iv. monitoring a position and/or displacement of the second closed electrically conducting loop, wherein
1. the third response is taken into account, and
2. the first and the second response are preferably not taken into account; and wherein
v. the third response is preferably not taken into account in monitoring the position and/or displacement of the first closed electrically conducting loop.
